(19) Europäisches Patentamt
European Patent Office
Office européen des brevets

(11) Numéro de publication: **0 117 959**
**B1**

(12) # FASCICULE DE BREVET EUROPÉEN

(45) Date de publication du fascicule du brevet: **03.09.86**

(51) Int. Cl.⁴: **C 07 F 9/38**, C 07 F 9/40, A 61 K 31/66

(21) Numéro de dépôt: **83402209.7**

(22) Date de dépôt: **16.11.83**

(54) **Nouveaux dérivés de la nitrosourée, leur procédé de préparation et les compositions pharmaceutiques les renfermant.**

(30) Priorité: **17.11.82 FR 8219199**

(43) Date de publication de la demande: **12.09.84 Bulletin 84/37**

(45) Mention de la délivrance du brevet: **03.09.86 Bulletin 86/36**

(84) Etats contractants désignés: **AT BE CH DE FR GB IT LI LU NL SE**

(56) Documents cité:
**US-A-3 920 733**

**ARCHIV DER PHARMAZIE, vol. 314, no. 11, 1981, pages 910-917, Verlag Chemie GmbH, Weinheim, DE; WEI-CI TANG et al.: "Synthesis of potentially antineoplastic derivatives of N- N-(2-chloroethyl)-N-nitrosocarbamoyl amino acids"**

(73) Titulaire: **ADIR, 22, rue Garnier, F-92200 Neuilly- sur-Seine (FR)**

(72) Inventeur: **Lavielle, Gilbert, 1 avenue Lily, F-78170 La Celle Saint Cloud (FR)**
Inventeur: **Cudennec, Claude, Dr., Avenue de Circout, F-78170 La Celle Saint Cloud (FR)**

(74) Mandataire: **Reverbori, Marcelle, ADIR 22 Rue Garnier, F-92200 Neuilly- sur- Seine (FR)**

**EP 0 117 959 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

## Description

La présente invention a pour objet de nouveaux dérivés de la nitrosourée, leur procédé de préparation et les compositions pharmaceutiques les renfermant.

Elle concerne particulièrement les dérivés de la nitrosourée de formule générale I:

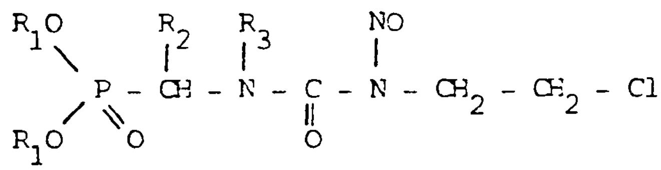

(I)

dans laquelle:

$R_1$ représente un atome d'hydrogène, un radical alkyle en chaine droite ou ramifiée renfermant de 1 à 6 atomes de carbone, ou un radical phényle pouvant porter comme substituant un atome d'halogène, un radical alkyle ou alcoxy renfermant de 1 à 5 atomes de carbone;

$R_2$ représente un atome d'hydrogène, un radical alkyle en chaine droite ou ramifiée renfermant de 1 à 6 atomes de carbone, et pouvant porter un radical carboxy ou alcoxycarbonyle renfermant de 2 à 6 atomes de carbone, un radical thiényle, phényle ou benzyle pouvant porter comme substituant un atome d'halogène, un radical alkyle ou alcoxy renfermant chacun de 1 à 5 atomes de carbone,

$R_3$ représente un atome d'hydrogène, un radical alkyle en chaine linéaire ou ramifiée ayant de 1 à 6 atomes de carbone ou un radical phényle ou benzyle pouvant porter comme substituant un atome d'halogène, un radical alkyle ou alcoxy ayant chacun de 1 à 5 atomes de carbone, ou bien $R_2$ et $R_3$ représentent ensemble un groupe $-(CH_2)_m-$dans lequel m prend les valeurs 3 ou 4,

sous forme racémique ou d'isomères optiques.

La présente invention a également pour objet le procédé de préparation des composés de formule générale I, caractérisé en ce que l'on condense un dérivé α-aminophosphoné de formule générale II

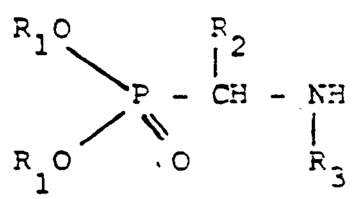

(II)

dans laquelle $R_1$, $R_2$ et $R_3$ ont les significations précédemment définies pour la formule I,

avec un excès de β-chloroethyl-isocyanate de formule III

$Cl = CH_2- CH_2 - N = C = O$ (III)

pour conduire à des dérivés nouveaux de formule générale IV:

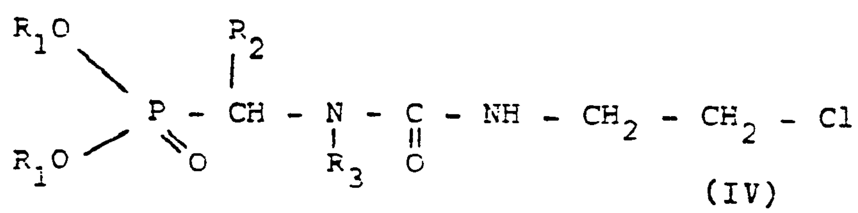

(IV)

(IV)

dans laquelle $R_1$, $R_2$ et $R_3$ ont les significations précédemment définies, et on nitrose les dérivés de formule IV pour obtenir les composés de formule I.

La condensation s'effectue de preférence dans l'eau ou dans un solvant choisi parmi les solvants chlorés tel que par exemple le chloroforme. Il est avantageux d'opérer à une température comprise entre 0 et 10°C et, éventuellement en présence d'une base minérale ou organique.

La nitrosation des nouveaux dérivés (IV) en nitrosourée de formule générale I est effectuée par exemple par l'action du nitrite de sodium dans l'acide formique refroidi à 5°C, ou bien encore par le chlorure de nitrosyle dans un solvant tel que la pyridine pendant plusieurs heures à la température de 0°C, selon les methodes décrites par J.L. MONTERO et Coll. dans Eur. J. Med. Chem. 1981, 16, 539.

Les matières premières de formule générale II sont des acides α-aminophosphoniques ou des α-aminophosphonates déjà connus dans la littérature et dont les principales synthèses ont été décrites par:

- K.D. BERLIN, R.T. CLAUNCH et E.T. GAUDY dans J. Org. Chem. 1968, 33, 3090
- J. KOWALIK et P. MASTALERZ dans Synthesis 1981, 57
- J. OLEKSYSZYN et R. TYKA dans Tetrahedron Letters 1977, 22, 2823
- et par A. DEHNEL et G. LAVIELLE dans Bull. Soc. Chim. Fr. 1978, 95.

Tous les dérivés nouveaux faisant partie de cette invention peuvent être purifiés par des méthodes physiques telles que cristallisation ou chromatographie.

La présente invention a également pour objet les isomères optiques des dérivés répondant à la formule générale I. Ces isomères peuvent être préparés à partir des formes optiquement actives de formule générale II par dédoublement des composés racémiques.

Comme agent de dédoublement, on peut citer par exemple, les acides (+) et (-) dibenzoyltartriques.

Les dérivés de formule générale I possèdent des propriétés pharmacologiques et thérapeutiques intéressantes. Ces derivés ont une activité antibactérienne et sont utiles pour le traitement des infections bactériennes chez l'homme et les animaux. En outre, les propriétés pharmacologiques de ces produits les rendent plus avantageux dans le traitement de la maladie cancéreuse que les nitrosourées prises comme référence et dont l'activité oncolytique et oncostatique est reconnus depuis longtemps.

Les composés selon l'invention sont testés par leur capacité à prolonger la survie de souris porteuses de cellules tumorales inoculées par voie intrapéritonéale ou intramusculaire conformément aux protocoles établis à l'Institut National du Cancer (U.S.A.) et publiés par R.I. GERAN et Coll. dans Cancer Chemotherapy Reports, 1972, part.III, Vol. 3(2), 1-87.

Il s'est révélé que les composés selon l'invention lorsqu'ils sont administrés par voie intrapéritonéale, sont capables de prolonger la survie des souris cancéreuses dès la dose de 1 mg/kg et d'induire des rémissions à long terme à la dose de 5 mg/kg comme c'est le cas pour le composé décrit dans l'exemple N°4. Administrés per os, ces composés augmentent la durée de vie des animaux cancéreux.

Le rapport doses actives per os sur dose active par voie intrapéritonéale est meilleur pour les composés selon l'invention que pour les produits de référence (F. SPREAFICO et Coll. dans "Nitrosoureas: current status and new developments"; A.W. PRESTAYKO et Coll. éditeurs, 1981, Academic Press (New York) Experimental Evaluation of Antitumor Drugs in the U.S.A. and U.S.S.R. and clinical correlations; National Cancer Institute Monograph N°55; National Institutes of Health U.S.A. 1980.)

Les composés selon l'invention peuvent freiner le développement des cellules cancéreuses inoculées intratécalement et prouvent ainsi leur faculté à franchir la barrière hématoencéphalique. De même, ces dérivés sont capables d'inhiber la croissance de carcinomes greffés dans le muscle ou sous la peau de souris même lorsque le traitement n'intervient que plusieurs jours après la greffe.

Le pouvoir antimétastasique des composés faisant l'objet de la présente invention est mesuré par la réduction, par rapport à des animaux non traités, du nombre et du poids des métastases pulmonaires développées après inoculation intramusculaire de cellules carcinomateuses. Les composés selon l'invention réduisent ou préviennent le développement des métastases, à titre d'exemple le composé n°4 empêche la formation des métastases lorsqu'il est administré à titre curatif à la dose de 10 mg/kg pendant 9 jours.

La toxicité hématopoiétique est évaluée, chez des animaux traités par une ou plusieurs administrations de composés selon l'invention, par le comptage des éléments figurés du sang périphérique et de la moelle osseuse, et la détection des cellules de repeuplement contenues dans la moelle osseuse (J.E. TILL et E.A. Mc CULLOCH, 1961, Radiation Res., 14, 213). Le nadir des concentrations cellulaires ainsi calculé est noté 3 jours après le début du traitement. L'intensité de cette dépression est mesurée après un traitement par une dose de N-N'-bis(2-chloroéthyl)-N-nitrosourée ("BCNU") - composé pris pour référence (W.C. TANG et G. EISENBRAND, Arch. Pharm. 1981, 314, 910) - connue pour avoir la meilleure efficacité contre les tumeurs cancéreuses, et par des doses de composés selon l'invention apportant le même bénéfice thérapeutique. Le retour à la formule sanguine normale s'opère dès le 10ème jour après le traitement par les composés de l'invention soit plus précocement qu'après administration de 25 mg/kg de BCNU. En outre, les composés nouveaux présentent une toxicité moins sévère à l'égard des cellules de renouvellement de la moelle osseuse.

La toxicité hépatique est évaluée notamment par la mesure selon Wroblewski de l'activité transaminase glutamique pyruvique contenue dans le sérum de rats Long Evans traités par une injection intrapéritonéale de 25 mg/kg de BCNU ou par des doses équipotentes des composés selon l'invention. A l'opposé du BCNU, les produits selon l'invention ne presentent pas de toxicité hépatique notoire. A titre d'exemple, le traitement par 25 mg/kg du composé N°4 n'entraîne pas d'élévation du taux de l'enzyme hors de la gamme habituellement rencontrée chez les rats non traités.

Les composés selon l'invention sont utiles en traitement oral ou parentéral chez l'homme dans les cas de leucémies carcinomes sarcomes, mélanomes, épithéliomes, gliomes, blastomes et, en général, de tumeurs cancéreuses de toutes localisations.

La présente invention a également pour objet les compositions pharmaceutiques contenant comme principe actif un dérivé de formule génerale I mélangé ou associé à un excipient pharmaceutiquement approprié.

Les compositions pharmaceutiques ainsi obtenues sont présentées avantageusement sous des formes diverses telles que par exemple comprimes, dragées, gélules, glossettes ou préparations galéniques appropriées pour une administration sublinguale, suppositoires, solutions injectables ou buvables.

Les exemples suivants, illustrent l'invention: Les points de fusion, sauf mention contraire, ont été déterminés à la plaque chauffante de KOFLER.

EXEMPLE 1: Acide [N-(chloro-2 ethyl) N-nitrosouréïdo] -1 éthyl phosphonique.

a) Préparation de l'acide [ N-(chloro-2 éthyl) uréïdo ] -1 éthylphosphonique:

Une solution de 2 g d'acide α-aminoéthyl phosphonique dans 20 ml d'eau est additionnée de 32 ml d'une solution de soude 1N, le milieu réactionnel étant refroidi à 0°C. On ajoute ensuite un équivalent (1,4 ml) d'isocyanate de chloroéthyle toujours à la même température. Après l'addition, l'agitation est poursuivie environ 30 minutes à température ambiante. Un nouvel équivalent d'isocyanate de β-chloroéthyle (1,4 ml) est ajouté et l'agitation est maintenue pendant 2 heures supplémentaires. A la fin de l'agitation, le précipité formé au cours de la réaction est séparé par filtration et la solution aqueuse est versée sur 30 ml de résine AG 50 W-X4 (copolymère sulfoné des styrène et divinylbenzène), récupérée et évaporée à sec. Le résidu est repris à sec par quelques millilitres d'alcool éthylique bouillant puis filtré à chaud ce qui permet d'isoler 0,3 g de produit de départ n'ayant pas réagi. Après évaporation de l'éthanol, le résidu obtenu est recristallisé dans l'eau.

Rendement: 54 % F = 180°C

Analyse:

C H N Cl

calculé 26,03 5,21 12,15 15,40

trouvé 26,32 5,22 12,20 15,14

b) Préparation de l'acide [N-(chloro-2 éthyl) N-nitrosouréïdo] -1 éthyl phosphonique.

Une solution contenant 1 g d'acide [N-(chloro-2 éthyl) uréïdo] -1 éthylphosphonique. précédemment obtenu dans 20 ml d'acide formique est refroidie à 0°C. On ajoute alors 1 g de nitrite de sodium par petites portions et après 30 minutes d'agitation, la solution est diluée avec 100 ml d'eau. La solution est passée sur 40 ml de résine AG 50 W-X4 (copolymère sulfoné des styrée et divinylbenzène) puis évaporée sous vide sans depasser la température de 30°C. L'huile jaune obtenue (1 g) est un peu instable et n'est pas purifiée davantage.

Elle est identifiée à l'acide [N-(chloro-2 éthyl) nitrosouréïdo] -1 éthyl phosphonique par les méthodes physiques habituelles (R.M.N.; I.R.: voir tableau).

EXEMPLE 2: Acide α-[ N-(chloro-2 éthyl) N-nitrosouréïdo] benzyl phosphonique.

L'acide α-[N-(chloro-2 éthyl) uréïdo] benzyl phosphonique est préparé par action de l'isocyanate de β-chloroéthyle et l'acide α-aminobenzyl phosphonique selon la méthode décrite dans l'exemple 1a.

Rendement: 75 %; F = 195°C

Analyse

C H N Cl

Calculé 49,33 5,87 10,28 8,69

Trouvé 49,03 5,70 10,26 8,22

L'acide α-[N-(chloro-2 éthyl) N-nitrosouréïdo] benzyl phosphonique est obtenu selon l'exemple 1b par action du nitrite de sodium sur l'acide-urée précédent.

Rendement: 56 % huile jaune légèrement instable.

Données physiques: Voir tableau.

EXEMPLE 3: Ester diéthylique de l'acide α-[N-(chloro-2 éthyl) N-nitrosouréïdo] benzyl phosphonique.

a) Préparation de l'ester diéthylique de l'acide α-[N-(chloro-2 éthyl) uréïdo] benzyl phosphonique.

Une solution 0.05 mole d'ester diéthylique de l'acide α-aminobenzyl phosphonique dans 30 ml de chloroforme est refroidie à 0°C dans un bain de glace puis additionnée par un large excès (0.06 mole) de β-chloroéthylisocyanate. La température de la réaction est ensuite maintenue à 10°C tout en poursuivant l'agitation jusqu'à la fin de réaction. (Des examens chromatographiques sur couche mince sont pratiques pour verifier la disparition complète de l'aminophosphonate de départ.) Après évaporation du solvant sous pression réduite, le résidu cristallin est repris à l'éther, filtré et donne 14,8 g de cristaux.

Rendement: 85 %; F = 82°C.

b) Préparation de l'ester diéthylique de l'acide α-[N-(chloro-2 éthyl) N-nitrosouréïdo] benzyl phosphonique.

L'urée obtenue précédemment (0,02 mole) est dissoute dans 60 ml d'acide formique et refroidie à 5°C. Un excès de nitrite de sodium (0,08 mole) est alors ajouté par petites portions pendant 1 heure.

Après évaporation sous vide de l'acide formique à une température inférieure à 35°C, le résidu est repris par 200 ml de dichlorométhane puis lavé 3 fois par 100 ml d'eau distillée. La phase organique est ensuite sechée sur sulfate de sodium, fitrée et évaporée sous pression réduite en laissant 5,5 g d'une huile qui est ensuite chromatographiée suivant la technique décrite par W.C. STILL dans J. Org. Chem. 1978, 43, 2923 en utilisant le mélange $CH_2Cl_2$-MeOH (99-1) comme éluant.

L'huile ainsi obtenu (4,7 g) cristallise dans de l'éther isopropylique en donnant des cristaux fondant à 95°C.

Rendement: 62 %.

**EXEMPLE 4 à 16:**

Les dérivés suivants ont été préparés selon le procédé décrit dans l'exemple 3:

4) Ester diéthylique de l'acide [N-(chloro-2 éthyl) N-nitrosouréïdo] -1 éthyl phosphonique; Rendement: 52 %; F = 85°C.

5) Ester diéthylique de l'acide α-[N-(chloro-2 éthyl) N-nitrosouréïdo] (chloro-4 benzyl) phosphonique; Rendement: 68 %;.F = 95°C.

6) Ester diéthylique de l'acide α-[N-(chloro-2 éthyl) N-nitrosouréïdo] (fluoro-4 benzyl) phosphonique;

Rendement: 74 %; F = 95°C.

7) Ester diéthylique de l'acide [N-(chloro-2 éthyl) N-nitrosouréïdo] -1 méthyl-2 propylphosphonique. Rendement: 68 %.

8) Ester diéthylique de l'acide [N-(chloro-2 éthyl) N-nitrosouréïdo] -1 butyl phosphonique: Rendement: 58 %.

9) Ester diéthylique de l'acide [N-(chloro-2 ethyl) N-nitrosouréïdo] -1 (méthoxy-4 phényl)-2 éthyl phosphonique: Rendement: 52 %; F = 98°C.

10) Ester diéthylique de l'acide [N-(chloro-2 éthyl) N-nitrosouréïdo] -1 phényl-2 éthyl phosphonique: Rendement: 46 %; F = 68°C.

11) Ester diéthylique de l'acide ([N-(chloro-2 éthyl) N-nitrosamidocarbonyl] -1 pyrrolidine) -2 phosphonique: Rendement: 48 %.

12) Ester diéthylique de l'acide [N-(chloro-2 éthyl) N-nitrosouréïdo] -1 (chloro-2 phényl) -2 éthyl phosphonique: Rendement: 62 %; F = 108°C.

13) Ester diéthylique de l'acide [N-(chloro-2 éthyl) N-nitrosouréïdo] (thiényl-2) méthyl phosphonique Rendement: 35 %; F = 70°C.

14) Ester diéthylique de l'acide α-[N-(chloro-2 éthyl) N-nitrosouréïdo] benzyl phosphonique; isomère lévogyre de l'exemple 3:

Rendement: 35 %; $\alpha^{22}{}_D$ = -34,4° (c = 2; $CHCl_3$).

15) Ester diéthylique de l'acide α-[N-(chloro-2 éthyl) N-nitrosouréïdo] benzyl phosphonique; isomère dextrogyre de l'exemple 3

Rendement 43 %; $\alpha^{22}{}_D$ = + 36° (c = 2; $CHCl_3$).

16) Ester diéthylique de l'acide [N-(chloro-2 éthyl) N-nitroso, N'-benzyluréïdo] méthyl phosphonique.

L'ester diéthylique de l'acide [N-(chloro-2 éthyl) N'-benzyl uréïdo] méthyl phosphonique de départ est préparé selon le procédé décrit dans l'exemple 1.

La nitrosation de l'urée précedente est effectuée au moyen de chlorure de nitrosyle selon la méthode suivante: On met en solution dans le dichlorométhane 0,02 moles d'urée obtenue selon le procédé décrit dans l'exemple 1 et après refroidissement à -10°C, on ajoute d'abord 7 ml de chlorure de nitrosyle puis 15 ml de pyridine. Après 2 heures d'agitation à 0°C, le mélange réactionnel est versé dans 250 ml d'eau glacée puis extrait par 3 portions de 100 ml de dichlorométhane. Les phases organiques réunies sont lavées d'abord par 2 portions de 50 ml d'acide chlorydrique à 5 % et enfin par de l'eau. Après séchage sur sulfate de sodium, la phase organique est évaporée sous pression réduite et laisse 7 g d'une huile qui est chromatographiée en utilisant le mélange chloroforme - acétate d'éthyle (95-5) comme éluant. Rendement: 70 %.

17) Ester diphénylique de l'acide [N-(chloro-2 éthyl) N-nitrosouréïdo] -1 éthylphosphonique: Rendement: 80 %; F = 84°C.

18) Ester di(méthoxy-2 phényl) de l'acide [N-(chloro-2 éthyl) N-nitrosouréïdo] -1 éthyl phosphonique: Rendement: 85 %; F = 83°C.

19) Ester diéthylique de l'acide [N-(chloro-2 éthyl) N-nitrosouréïdo] -1 carboxy-2 éthyl phosphonique; Rendement: 72 %; F = 90°C.

20) Ester diéthylique de l'acide [N-(chloro-2 éthyl) N-nitrosouréïdo] -1 éthoxycarbonyl-2 éthyl phosphonique. Rendement: 80 %; F = 103°C.

Les caractéristiques physiques des composés intermédiaires de formule générale IV sont décrites dans le tableau I suivant:

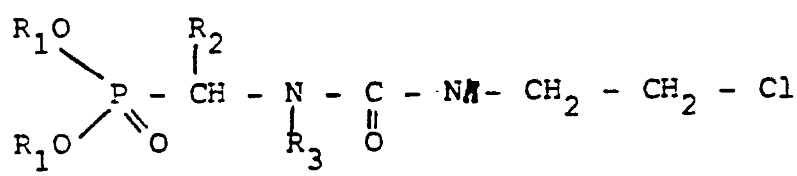

(IV)

TABLEAU I

| $R_1$ | $R_2$ | $R_3$ | Rdt % | F(°C) | I.R.($cm^{-1}$) ν(NH) | ν(CO) | R.M.N |
|---|---|---|---|---|---|---|---|
| $C_2H_5$ | 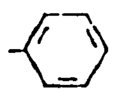 | H | 80 | 82 | 3380<br>3250 | 1665<br>1550 | 1,05,t,3H ; 1,35,t,3H ; 3,5,m,4H ;<br>3,8,q,2H ; 4,3,q,2H ; ($J_{PH}$=7Hz)<br>5,5,d de d,1H ($J_{PH}$=23Hz ; $J_{HH_5}$=10Hz)<br>H échangeable : 6,5,m,1H ; 7,5,m,1H ;<br>7,5,m,5H Ar |
| $C_2H_5$ | $-CH_3$ | H | 85 | <50 | 3200<br>3400 | 1650-<br>1690<br>1550 | 1,1 à 1,7,m,9H ;<br>3,6,m,4H ; 3,9 à 4,6,m,5H<br>2H échangeables ∼ 6,6 |
| $C_2H_5$ | 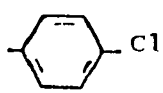 | H | 70 | 122 | 3370<br>3290 | 1680<br>1550 | 1,t,3H ; 1,5,t,3H ;<br>3,5,m,4H ; 3,7 à 4,6,m,4H ;<br>5,5,d,1H ; 6,45,s,1H échangeable<br>6,4 à 7,7,m,5H dont 1H échangeable. |
| $C_2H_5$ | 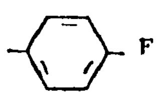 | H | 95 | 105 | 3300<br>3360 | 1685<br>1550 | 1,t,3H ; 1,5,t,3H ;<br>3,5,m,4H ; 3,6 à 4,5,m,4H<br>5,5,d,1H ; 6,4,s,1H échangeable<br>6,8 à7,7,m,5H |
| $C_2H_5$ | 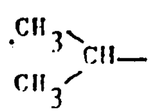 | H | 96 | 115 | 3350 | 1680<br>1550 | 0,9 à 1,6,m,12H ;<br>1,7 à 2,5,m,1H ;<br>3,6,m,4H ; 3,8 à 4,6,m,5H ;<br>6,6,m,2H échangeables |

TABLEAU I ( SUITE I)

| $R_1$ | $R_2$ | $R_3$ | Rdt % | F(°C) | I.R.($cm^{-1}$) $\nu$(NH) | $\nu$(CO) | R.M.N |
|---|---|---|---|---|---|---|---|
| $C_2H_5$ | $CH_3-(CH_2)_2-$ | H | 88 | - | 3350 | 1680<br>1550 | 0,7 à 1,7,m,13H ;<br>3,5,m,4H ;<br>3,8 à 4,3,m,5H<br>6,2 à 6,5,m,2H échangeables |
| $C_2H_5$ | 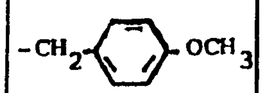 | H | 82 | - | | | 1,3,t,6H ; 2,7 à 3,m,3H ;<br>3,4,m,4H ; 3,6,s,3H ;<br>6,2,s,1H échangeable ; 6,5,d,1H<br>échangeable ; 6,6 à 7,1 ; spectr. AB,4H |
| $C_2H_5$ | 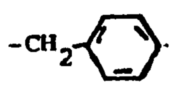 | H | 92 | 105 | | | 1,2,t,6H ; 2,7 à 3,1,m,3H ;<br>3,4,m,4H ; 3,8 à 4,2,m,4H<br>6,2,s,1H échangeable ; 6,5,d,1H<br>échangeable ; 7,1,s,5H Ar, |
| $C_2H_5$ | $-(CH_2)_3-$ | | 95 | - | 3320 | 1640<br>1535 | 1,3,t,6H ; 1,7 à 2,3,m,4H ;<br>3,2 à 3,6,m,6H<br>3,7 à 4,3,m,5H<br>6,5,s,1H échangeable |
| $C_2H_5$ | 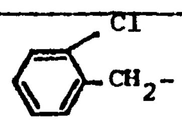 | H | 86 | 97 | | | |
| $C_2H_5$ | 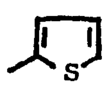 | H | 95 | 64 | | | 1 à 1,4,m,6H ; 3,45,m,4H ;<br>3,8 à 4,3,m,4H ;<br>5,65,d de d,1H ;<br>6,4,s,1H échangeable ;<br>6,7 à 7,2,m,4H. |

TABLEAU I (SUITE 2)

| $R_1$ | $R_2$ | $R_3$ | Rdt % | F(°C) | I.R.($cm^{-1}$) $\nu$(NH) | $\nu$(CO) | R.M.N |
|---|---|---|---|---|---|---|---|
| $-C_2H_5$ | 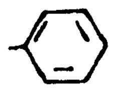 | H | 90 | - | | | C = 2 éthanol : Pouvoir rotatoire $\alpha_D^{22}$ = + 9,5° Autres caractéristiques voir premier exemple de ce tableau |
| $-C_2H_5$ | 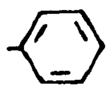 | H | 90 | - | | | C = 2 éthanol : Pouvoir rotatoire $\alpha_D^{22}$ = - 9,3° Autres caractéristiques voir premier exemple de ce tableau |
| $-C_2H_5$ | H | $CH_2$ 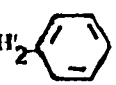 | 85 | 64 | 3310 | 1640 1540 | 1,3,t,6H ; 3,4, à 3,6,m,6H ; 3,8 à 4,3,m,4H ; 4,5,s,2H ; 5,8,s,1H échangeable ; 7,2,s,5H |
| 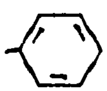 | $-CH_3$ | H | 82 | 98 | 3340 | 1690 1555 | 1,3,d, de d,3H ($J_{PH}$=17Hz, $J_{HH}$=6,7Hz 3,3,m,4H ; 4,2 à 4,7,m,1H ; 5,8,s,1H échangeable ; 6,15,d,1H échangeable ; 6,8 à 7,2,m,10H. |
| 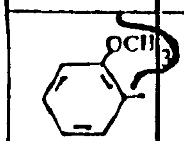 | $-CH_3$ | H | 86 | | | | 1,3,d de d, 3H ; 3,3,s,4H ; 3,5,s,3H ; 3,6,s,3H ; 4,2 à 4,8,m,1H ; 5,8 à 6,3,m,2H échangeables. 6,5 à 7,1,m,8H. |

TABLEAU I (SUITE 3)

| $R_1$ | $R_2$ | $R_3$ | Rdt % | F(°C) | I.R.($cm^{-1}$) ν(NH) | ν(CO) | R.M.N |
|---|---|---|---|---|---|---|---|
| $C_2H_5$ | $-CH_2COOC_2H_5$ | H | 70 | 67 | 3380<br>3440 | ester<br>1740<br>I. 1630<br>II. 1570 | 1,1 à 1,5,m,9H ; 2,5 à 2,7,m,2H ; 3,5,m,4H ; 3,8 à 4,2,m,6H ; 4,2 à 4,9,m,1H ; 6,3 à 6,5,m,2H échangeables. |
| $C_2H_5$ | $-CH_2COOH$ | H | 55 | 144 | 3260<br>3360 | 1720<br>1630<br>1570 | 1,2 à 1,4,t,6H ; 2,3 à 2,7,m,2H 3,3 à 3,8,m,4H ; 3,8 à 4,5,m,4H ; 4 à 5,m,1H ;<br>Spectre réalisé dans $D_2O$ + $DM_{SO}$. |

Les caractéristiques physiques des composé de formule générale I sont décrites dans le tableau suivant:

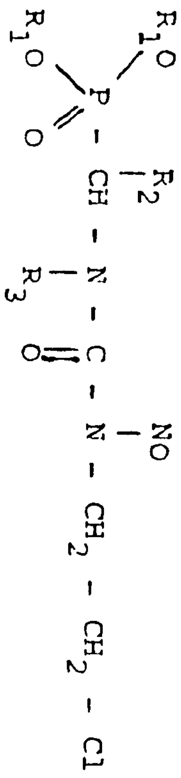

I

TABLEAU II

| EX. | $R_1$ | $R_2$ | $R_3$ | I.R. ($cm^{-1}$) ν(NH) | ν(CO) | R.M.N |
|---|---|---|---|---|---|---|
| 1 | -H | $-CH_3$ | H | | | 1,10 à 1,50,d de d ($J_{PH}$=15,4Hz, $J_{HH}$=7Hz),3H ; 3,50,t, (J=6Hz),2H ; 4,10,t,2H ; 3,70 à 4,30,m,1H |
| 2 | -H | 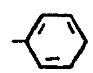 | H | | | 3,40,t,2H ; 3,95,t,2H ; 5,10,d, ($J_{PH}$=20Hz),1H ; 7,20,m,5H. |
| 3 | $-C_2H_5$ | 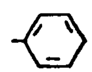 | H | 3180 | 1720 | 1,10,t,3H ; 1,25,t,3H ; 3,45,m,2H ; 3,7 à 4,5,m, 6H ; 5,55 d de d, ($J_{PH}$=21Hz et $J_{HH}$=9Hz), 1H ; 6,9 à 8,1,m,6H. |
| 4 | $-C_2H_5$ | $-CH_3$ | H | 3220 | 1720 | 1,20 à 1,80,9H ; 3,40 à 3,70,m,2H ; 3,90 à 5,00,m,5H ; 7,40,m,1H échangeable |
| 5 | $-C_2H_5$ | 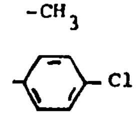 | H | 3180 | 1710 | 1,20,m,6H ; 3,45,t,2H ; 3,70 à 4,40,m,6H ; 5,50 d de d,1H ; 7,40,s,4H ; 7,80,m,1H échangeable. |
| 6 | $-C_2H_5$ | 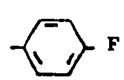 | H | 3180 | 1715 | 1,00 à 1,50,m,6H ; 3,30 à 4,40,m,8H ; 5,50,m,1H ; 6,90 à 8,00,m,5H. |
| 7 | $-C_2H_5$ | $-CH(CH_3)CH_3$ | H | 3220 | 1720 | 1,00 à 1,50,m,12H ; 1,90 à 2,60,m,1H ; 3,50,t,2H ; 3,90 à 4,70,m,5H ; 7,20,m,1H échangeable. |
| 8 | $-C_2H_5$ | $-(CH_2)_2-CH_3$ | H | 3220 | 1720 | 0,90 à 2,10,m,13H ; 3,50,t,2H ; 3,90 à 4,50,m,6H ;4,50 à 5,00,m,1H 7,30,m,1H échangeable. |

TABLEAU II ( SUITE 1)

| EX. | $R_1$ | $R_2$ | $R_3$ | I.R. ($cm^{-1}$) ν(NH) | ν(CO) | R.M.N |
|---|---|---|---|---|---|---|
| 9 | $-C_2H_5$ | 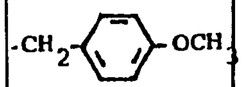 | H | 3230 | 1710 | 1,10,t,3H ; 1,50,t,3H ; 2,80 à 3,50,m,4H 3,90 à 4,50,m,6H ; 3,75,s,3H ; 4,50 à 5,00,m,1H ; 6,70 à 7,30,m,4H ; 7,40,m,1H échangeable. |
| 10 | $-C_2H_5$ | 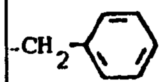 | H | 3240 | 1720 | 1,10;t;3H ; 1,50,t,3H ; 3,00 à 3,50,m,4H 3,90 à 4,50,m,6H ; 4,50 à 5,00,m,1H ; 7,30,s,5H ; 7,60,m,1H échangeable. |
| 11 | $-C_2H_5$ | $-(CH_2)_3-$ | | | 1690 | 1,10 à 1,50,m,6H ; 1,80 à 2,70,m,4H ; 3,40 à 4,00,m,4H ; 4,00 à 4,50,m,6H ; 4,80,m,1H. |
| 12 | $-C_2H_5$ | 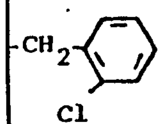 | H | 3240 | 1725 | 1,20 à 1,60,m,6H ; 3,00 à 3,50,m,4H ; 3,50 à 4,50,m,6H ; 4,50 à 5,20,m,1H ; 7,00 à 7,50,m,4H ; 7,50,m,1H échangeable. |
| 13 | $-C_2H_5$ | 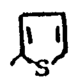 | H | 3170 | 1720 | 1,00 à 1,50,2t,6H ; 3,40 à 3,60,t,2H ; 3,90 à 4,50,m,6H ; 6,90 à 7,50,m,3H ; 7,60 à 7,90,m,1H échangeable. |
| 14 | $-C_2H_5$ | 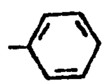 | H | | | Isomère lévrogyre $\alpha_D^{22}$ = -34,4° (C=2 ; $CHCl_3$) I.R. et R.M.N. Idem Exemple 3 |
| 15 | $-C_2H_5$ | 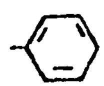 | H | | | Isomère dextrogyre $\alpha_D^{22}$ = +36°C (C=2 ; $CHCl_3$) I.R. et R.M.N. Idem Exemple 3 |

0117959

TABLEAU II (SUITE 2)

| EX. | $R_1$ | $R_2$ | $R_3$ | I.R. ($cm^{-1}$) ν(NH) | ν(CO) | R.M.N |
|---|---|---|---|---|---|---|
| 16 | $-C_2H_5$ | -H | $-CH_2$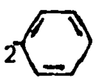 | | 1690 | 1,20,t,3H ; 1,40,t,3H ; 3,30 à 4,50,m,10H<br>4,90,s,2H ; 7,40,s,5H. |
| 17 | $-C_6H_5$ | $-CH_3$ | H | 3220 | 1710 | 1,60,d de d, ($J_{PH}$=17z, $J_{HH}$=7Hz),3H<br>3,35,t,($J_{HH}$=6Hz),2H ; 4,00,t,(J=6Hz),2H ;<br>4,75,m,1H ; 7,s,10H ; 7,35,d,1H échangeable |
| 18 | $-H_3CO$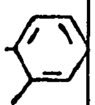 | $-CH_3$ | H | 3380 | 1730 | 1,4 à 1,9,d de d,3H ; 3,3 à 3,6,t,2H ;<br>3,8,2s,6H ; 4 à 4,3,t,2H ; 4,5 à 5,5,5m,1H ;<br>6,8 à 7,4,m,8H ; 8,s,1H échangeable. |
| 19 | $-C_2H_5$ | $-CH_2COOC_2H_5$ | H | 3240 | 1740<br>1720<br>1520 | 1,1 à 1,5,m,9H ; 2,7 à 3,1,m,2H ;<br>3,4 à 3,7,m,2H ; 3,9 à 4,5,m,6H ;<br>4,5 à 5,5,m,1H ; 7,7 à 7,8,s,1H échangeable. |
| 20 | $-C_2H_5$ | $-CH_2COOH$ | H | 3280 | 1700<br>1710<br>1535 | 1,3 à 1,6,2t,6H ; 2,8 à 3,3,m,2H ;<br>3,4 à 3,6,m,2H ; 4 à 4,6,m,4H ; 4,5 à 5,5,m,1H. |

**Revendications** (BE → SE)

1. Dérivés de la nitrosourée répondant à la formule générale I:

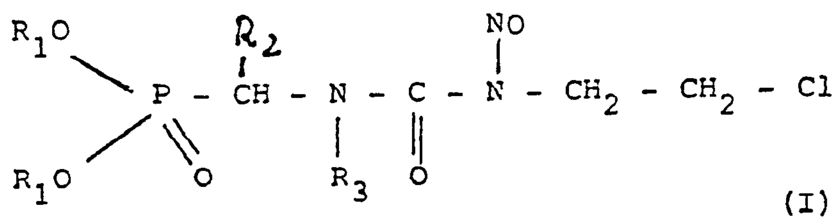

(I)

dans laquelle:

$R_1$ représente un atome d'hydrogène, un radical alkyle en chaîne droite ou ramifiée de 1 à 6 atomes de carbone, ou un radical phényle pouvant porter comme substituant un atome d'halogène un radical alkyle ou alcoxy renfermant de 1 à 5 atomes de carbone;

$R_2$ représente un atome d'hydrogène, un radical alkyle en chaîne droite ou ramifiée de 1 à 6 atomes de carbone, et pouvant porter un radical carboxy ou alcoxycarbonyle renfermant de 2 à 6 atomes de carbone, un radical thiényle, phényle ou benzyle pouvant porter comme substituant un atome d'halogène, un radical alkyle ou alcoxy renfermant de 1 à 5 atomes de carbone;

$R_3$ représente un atome d'hydrogène, un radical alkyle en chaîne linéaire ou ramifiée de 1 à 6 atomes de carbone, ou un radical phényle ou benzyle pouvant porter comme substituant un atome d'halogène, un radical alkyle ou alcoxy ayant chacun de 1 à 5 atomes de carbone, ou bien

$R_2$ et $R_3$ représentent ensemble un groupe $-(CH_2)_m-$dans lequel m prend les valeurs 3 ou 4,

sous forme racémique ou isomères optiques.

2. L'ester diéthylique de l'acide α-[N-(chloro-2 éthyl) N-nitrosouréïdo] benzylphosphonique.

3. L'ester diéthylique de l'acide [N-(chloro-2 éthyl) N-nitrosouréïdo] -1 éthylphosphonique.

4. L'ester diphénylique de l'acide [N-(chloro-2 éthyl) N-nitrosouréïdo] -1 éthyl phosphonique.

5. Ester diéthylique de l'acide [N-(chloro-2 éthyl) N-nitrosouréïdo] -1 éthoxycarbonyl-2 éthyl phosphonique.

6. Procédé de préparation des composés de la revendication 1, caractérisé en ce que l'on condense un dérivé α-aminophosphone de formule générale II:

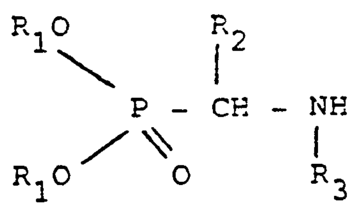

(II)

dans laquelle $R_1$, $R_2$ et $R_3$ ont les significations définies dans la revendication 1,
avec un excès de β-chloroéthylisocyanate de formule III:

$Cl - CH_2 - CH_2 - N = C = 0$ (III)

pour conduire à des dérivés de formule générale IV:

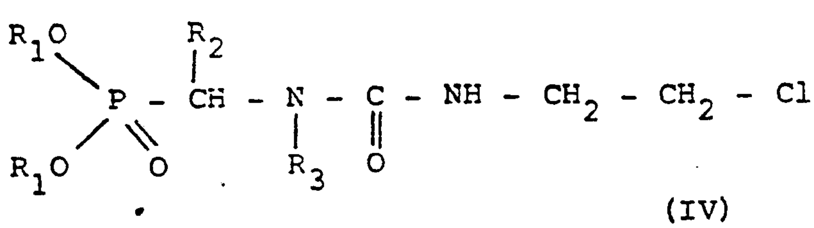

(IV)

dans laquelle $R_1$, $R_2$ et $R_3$ ont les significations définies dans la revendication 1, qui sont ensuite nitrosés par un agent nitrosant.

7. Procédé selon la revendication 6 caractérisé en ce que les composés de formule générale II sont condensés avec le β-chloroéthylisocyanate III dans l'eau ou dans un solvant chloré à une température comprise entre 0 et 10°0 et en présence ou en l'absence d'une base organique ou minérale.

8. Procédé selon la revendication 6 caractérisé en ce que les composés de formule générale IV sont nitrosés en composés de formule générale I au moyen de nitrite de sodium dans l'acide formique ou par le chlorure de nitrosyle dans la pyridine.

9. Compositions pharmaceutiques contenant comme principe actif un composé selon l'une quelconque des

revendications 1 à 5, en association ou en mélange avec un excipient ou un véhicule inerte non toxique; pharmaceutiquement acceptable.

10. Composés répondant à la formule générale IV:

FIG16/40 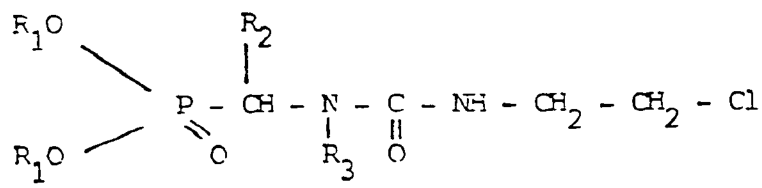 (IV)

dans laquelle les symboles $R_1$, $R_2$ et $R_3$ ont la même signification que dans la revendication 1,

**Revendications** (AT)

1. Procédé de préparation de dérivés de la nitrosourée répondant à la formule générale I:

FIG17/40 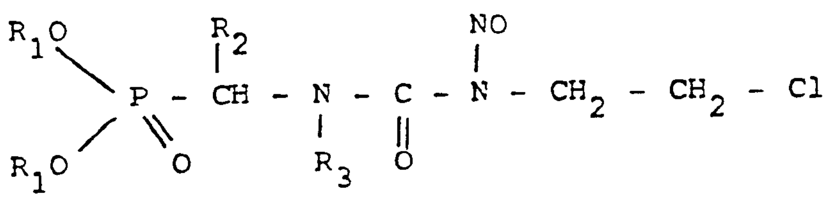 (I)

dans laquelle:

$R_1$ représente un atome d'hydrogène, un radical alkyle en chaîne droite ou ramifiée de 1 à 6 atomes de carbone, ou un radical phényle pouvant porter comme substituant un atome d'halogène un radical alkyle ou alcoxy renfermant de 1 à 5 atomes de carbone.

$R_2$ représente un atome d'hydrogène, un radical alkyle en chaîne droite ou ramifiée de 1 à 6 atomes de carbone, et pouvant porter un radical carboxy ou alcoxycarbonyle renfermant de 2 à 6 atomes de carbone, un radical thiényle, phényle ou benzyle pouvant porter comme substituant un atome d'halogène, un radical alkyle ou alcoxy renfermant de 1 à 5 atomes de carbone,

$R_3$ représente un atome d'hydrogène, un radical alkyle en chaîne linéaire ou ramifiée de 1 à 6 atomes de carbone, ou un radical phényle ou benzyle pouvant porter comme substituant un atome d'halogène, un radical alkyle ou alcoxy ayant chacun de 1 à 5 atomes de carbone, ou bien

$R_2$ et $R_3$ représentent ensemble un groupe $-(CH_2)_m$ dans lequel m prend les valeurs 3 ou 4, sous forme racémique ou isomères optiques,

caractérisé en ce que l'on condense un derive α-aminophosphoné de formule générale II:

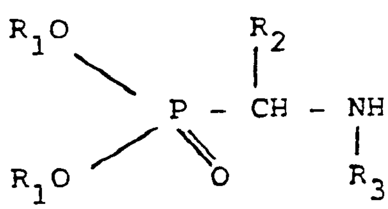 (II)

dans laquelle $R_1$, $R_2$ et $R_3$ ont les significations définies dans la formule I
avec un excès de β-chloroéthylisocyanate de formule III:
$Cl-CH_2-CH_2-N=C=O$ (III)
pour conduire à des dérives de formule générale IV:

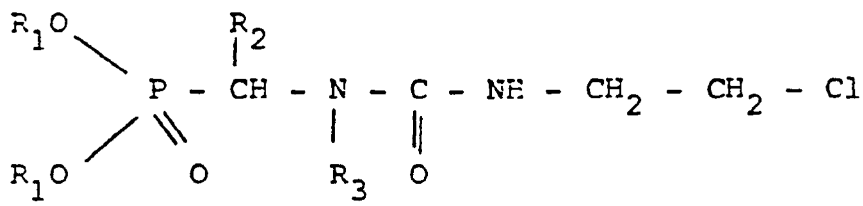

(IV)

dans laquelle $R_1$, $R_2$ et $R_3$ ont les significations définies dans la formule I, qui sont ensuite nitrosés par un agent nitrosant.

2. Procédé selon la revendication 1 caractérisé en ce que les composés de formule générale II sont condensés avec le β-chloroéthylisocyanate III dans l'eau ou dans un solvant chloré à une température comprise entre 0 et 10°C et en présence ou en l'absence d'une base organique ou minérale.

3. Procédé selon la revendication 1 caractérisé en ce que les composés de formule générale IV sont nitrosés en composés de formule générale I au moyen de nitrite de sodium dans l'acide formique ou par le chlorure de nitrosyle dans la pyridine.

**Patentansprüche**

für den Vertragsstaat AT

1. Verfahren zur Herstellung von Nitrosoharnstoffderivaten der allgemeinen Formel I

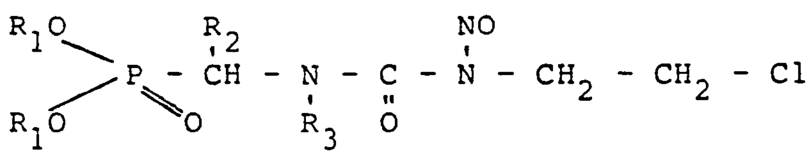

(I)

in der

$R_1$ ein Wasserstoffatom, eine geradkettige oder verzweigte Alkylgruppe mit 1 bis 6 Kohlenstoffatomen oder eine Phenylgruppe, die als Substituenten ein Halogenatom, eine Alkylgruppe oder eine Alkoxygruppe mit 1 bis 5 Kohlenstoffatomen tragen kann;

$R_2$ ein Wasserstoffatom, eine geradkettige oder verzweigte Alkylgruppe mit 1 bis 6 Kohlenstoffatomen, die eine Carboxygruppe oder eine Alkoxycarbonylgruppe mit 2 bis 6 Kohlenstoffatomen tragen kann, oder eine Thienyl-, Phenyl- oder Benzyl-Gruppe, die als Substituenten ein Halogenatom, eine Alkylgruppe oder eine Alkoxygruppe mit 1 bis 5 Kohlenstoffatomen tragen kann;

$R_3$ ein Wasserstoffatom, eine geradkettige oder verzweigte Alkylgruppe mit 1 bis 6 Kohlenstoffatomen oder eine Phenyl- oder Benzyl-Gruppe, die als Substituent ein Halogenatom, eine Alkylgruppe oder eine Alkoxygruppe mit jeweils 1 bis 5 Kohlenstoffatomen tragen kann, oder

$R_2$ und $R_3$ gemeinsam eine Gruppe der Formel $-(CH_2)_m-$, in der m die Werte 3 oder 4 besitzt, bedeuten,

in Form des Racemats oder der optischen Isomeren, dadurch gekennzeichnet, daß man ein α-Aminophosphonsäurederivat der allgemeinen Formel II

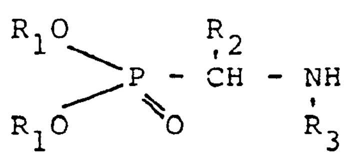

(II)

in der $R_1$, $R_2$ und $R_3$ die bezüglich der allgemeinen Formel I angegebenen Bedeutungen besitzen, mit einem Überschuß des β-Chlorethylisocyanats der Formel III

$Cl-CH_2-CH_2-N=C=O$ (III)

kondensiert zur Bildung der Derivate der allgemeinen Formel IV

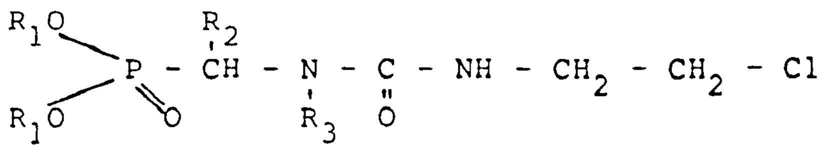 (IV)

in der $R_1$, $R_2$ und $R_3$ die bezüglich der allgemeinen Formel I angegebenen Bedeutungen besitzen, die anschließend mit einem Nitrosierungsmittel nitrosiert werden.

2. Verfahren nach Anspruch 1, dadurch gekennzeichnet, daß die Verbindungen der allgemeinen Formel II in Wasser oder in einem chlorierten Lösungsmittel bei einer Temperatur zwischen 0 und 10°C und in Gegenwart oder in Abwesenheit einer organischen oder anorganischen Base mit dem β-Chlorethylisocyanat der Formel III kondensiert werden.

3. Verfahren nach Anspruch 1, dadurch gekennzeichnet, daß die Verbindungen der allgemeinen Formel IV mit Natriumnitrit in Ameisensäure oder mit Nitrosylchlorid in Pyridin zu den Verbindungen der allgemeinen Formel I nitrosiert werden.

**Patentansprüche**

für die Vertragsstaaten BE, CH, DE, FR, GB, IT, LI, LU, NL, SE)

1. Nitrosoharnstoffderivate der allgemeinen Formel I

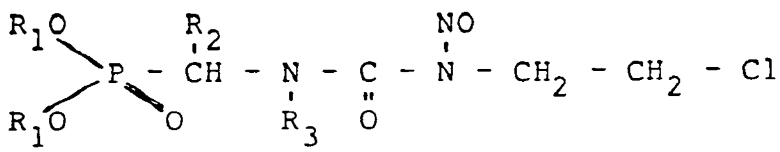 (I)

in der

$R_1$ ein Wasserstoffatom, eine geradkettige oder verzweigte Alkylgruppe mit 1 bis 6 Kohlenstoffatomen oder eine Phenylgruppe, die als Substituenten ein Halogenatom, eine Alkylgruppe oder eine Alkoxygruppe mit 1 bis 5 Kohlenstoffatomen tragen kann;

$R_2$ ein Wasserstoffatom, eine geradkettige oder verzweigte Alkylgruppe mit 1 bis 6 Kohlenstoffatomen, die eine Carboxygruppe oder eine Alkoxycarbonylgruppe mit 2 bis 6 Kohlenstoffatomen tragen kann, oder eine Thienyl-, Phenyl- oder Benzyl-Gruppe, die als Substituenten ein Halogenatom, eine Alkylgruppe oder eine Alkoxygruppe mit 1 bis 5 Kohlenstoffatomen tragen kann;

$R_3$ ein Wasserstoffatom, eine geradkettige oder verzweigte Alkylgruppe mit 1 bis 6 Kohlenstoffatomen oder eine Phenyl- oder Benzyl-Gruppe, die als Substituent ein Halogenatom, eine Alkylgruppe oder eine Alkoxygruppe mit jeweils 1 bis 5 Kohlenstoffatomen tragen kann, oder

$R_2$ und $R_3$ gemeinsam eine Gruppe der Formel $-(CH_2)_m-$, in der m die Werte 3 oder 4 besitzt, bedeuten, in Form des Racemats oder der optischen Isomeren.

2. Diethylester der α-[N-(2-Chlor-ethyl)-N-nitroso-ureido]-benzylphosphonsäure.

3. Diethylester der 1-[N-(2-Chlor-ethyl)-N-nitroso-ureido]-ethylphosphonsäure.

4. Diphenylester der 1-[N-(2-Chlor-ethyl)-N-nitroso-ureido]-ethylphosphonsäure.

5. Diethylester der 1-[N-(2-Chlor-ethyl)-N-nitroso-ureido]-2-ethoxycarbonyl-ethylphosphonsäure.

6. Verfahren zur Herstellung der Verbindungen nach Anspruch 1, dadurch gekennzeichnet, daß man ein q-Aminophosphonsäurederivat der allgemeinen Formel II

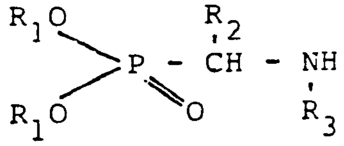 (II)

in der $R_1$, $R_2$ und $R_3$ die in Anspruch 1 angegebenen Bedeutungen besitzen, mit einem Überschuß des β-Chlorethylisocyanats der Formel III
$Cl - CH_2 - CH_2 - N = C = 0$ (III)
kondensiert zur Bildung der Derivate der allgemeinen Formel IV

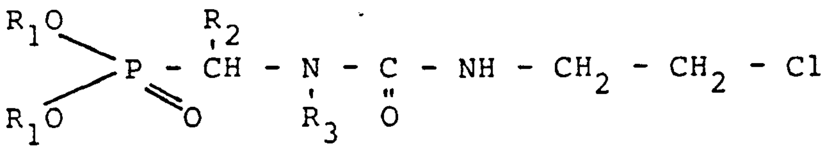 (IV)

in der $R_1$, $R_2$ und $R_3$ die in Anspruch 1 angegebenen Bedeutungen besitzen, die anschließend mit einem Nitrosierungsmittel nitrosiert werden.

7. Verfahren nach Anspruch 6, dadurch gekennzeichnet, daß die Verbindungen der allgemeinen Formel II in Wasser oder in einem chlorierten Lösungsmittel bei einer Temperatur zwischen 0 und 10°C und in Gegenwart oder in Abwesenheit einer organischen oder anorganischen Base mit dem β-Chlorethylisocyanat der Formel III kondensiert werden.

8. Verfahren nach Anspruch 6, dadurch gekennzeichnet, daß die Verbindungen der allgemeinen Formel IV mit Natriumnitrit in Ameisensäure oder mit Nitrosylchlorid in Pyridin zu den Verbindungen der allgemeinen Formel I nitrosiert werden.

9. Pharmazeutische Zubereitungen enthaltend als Wirkstoff eine Verbindung nach einem der Ansprüche 1 bis 5 in Kombination oder in Mischung mit einem inerten oder nichttoxischen, pharmazeutisch annehmbaren Hilfsmittel oder Trägermaterial.

10. Verbindungen der allgemeinen Formel IV

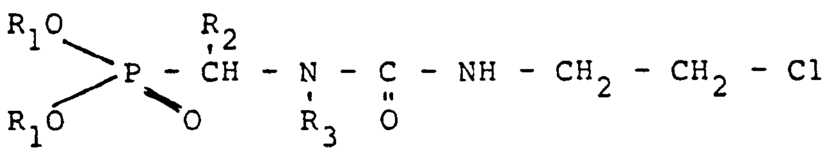 (IV)

in der die Symbole $R_1$, $R_2$ und $R_3$ die in Anspruch 1 angegebenen Bedeutungen besitzen.

**Claims** for the Contracting State AT

1. Process for the preparation of nitrosourea derivatives corresponding to the general formula I:

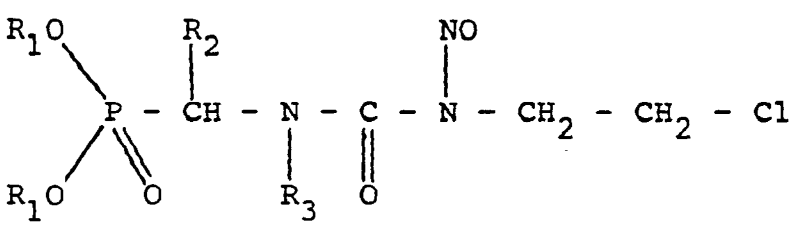 (I)

in which:

$R_1$ represents a hydrogen atom, a straight-chain or branched alkyl radical having from 1 to 6 carbon atoms, or a phenyl radical that may be substituted by a halogen atom or an alkyl or alkoxy radical containing from 1 to 5 carbon atoms;

$R_2$ represents a hydrogen atom, a straight-chain or branched alkyl radical having from 1 to 6 carbon atoms that may be substituted by a carboxy or alkoxycarbonyl radical containing from 2 to 6 carbon atoms, a thienyl, phenyl or benzyl radical that may be substituted by a halogen atom or an alkyl or alkoxy radical containing from 1 to 5 carbon atoms;

$R_3$ represents a hydrogen atom, a straight-chain or branched alkyl radical having from 1 to 6 carbon atoms, or a phenyl or benzyl radical that may be substituted by a halogen atom or an alkyl or alkoxy radical, each containing from 1 to 5 carbon atoms, or alternatively

$R_2$ and $R_3$ together represent a group $-(CH_2)_m-$ in which m is 3 or 4,

in the racemic form or as optical isomers, characterised in that an α-aminophosphonate derivative of the general formula II:

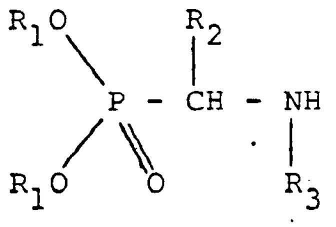

(II)

in which $R_1$, $R_2$ and $R_3$ have the meanings defined in formula I, is condensed with an excess of β-chloroethyl isocyanate of the formula III:

$Cl-CH_2-CH_2-N=C=O$ (III)

to give derivatives of the general formula IV:

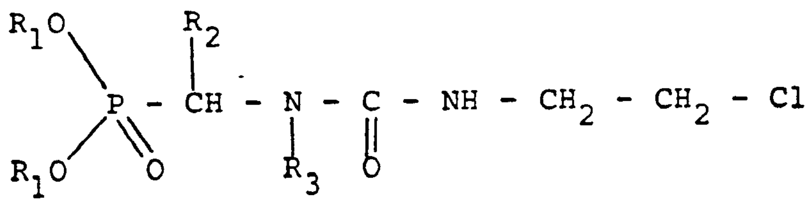

(IV)

in which $R_1$, $R_2$ and $R_3$ have the meanings defined in formula I, which derivatives are then converted into nitroso compounds by means of a nitrosation agent.

2. Process according to claim 1, characterised in that the compounds of the general formula II are condensed with β-chloroethyl isocyanate III in water or in a chlorinated solvent at a temperature of from 0 to 10°C and in the presence or absence of an organic or mineral base.

3. Process according to claim 1, characterised in that the compounds of the general formula IV are converted into nitroso compounds of the general formula I by means of sodium nitrite in formic acid or by means of nitrosyl chloride in pyridine.

**Claims** for the Contracting States BE, CH, DE, FR, GB,

IT, LI, LU, NL, SE

1. Nitrosourea derivatives corresponding to the general formula I:

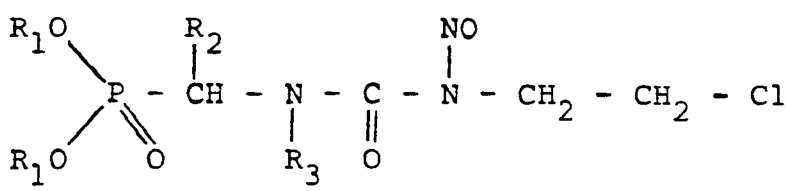

(I)

in which:

$R_1$ represents a hydrogen atom, a straight-chain or branched alkyl radical having from 1 to 6 carbon atoms, or a phenyl radical that may be substituted by a halogen atom or an alkyl or alkoxy radical containing from 1 to 5 carbon atoms;

$R_2$ represents a hydrogen atom, a straight-chain or branched alkyl radical having from 1 to 6 carbon atoms that may be substituted by a carboxy or alkoxycarbonyl radical containing from 2 to 6 carbon atoms, a thienyl, phenyl or benzyl radical that may be substituted by a halogen atom or an alkyl or alkoxy radical containing from 1 to 5 carbon atoms;

$R_3$ represents a hydrogen atom, a straight-chain or branched alkyl radical having from 1 to 6 carbon atoms, or a phenyl or benzyl radical that may be substituted by a halogen atom or an alkyl or alkoxy radical, each containing from 1 to 5 carbon atoms, or alternatively

$R_2$ and $R_3$ together represent a group $-(CH_2)_m-$in which m is 3 or 4, in the racemic form or as optical isomers.

2. The diethyl ester of α-[N-(2-chloroethyl)-N-nitrosoureido]-benzylphosphonic acid.

3. The diethyl ester of 1-[N-(2-chloroethyl)-N-nitrosoureido]-ethylphosphonic acid.

4. The diphenyl ester of 1-[N-(2-chloroethyl)-N-nitrosoureido]-ethylphosphonic acid.

5. The diethyl ester of 1-[N-(2-chloroethyl)-N-nitrosoureido]-2-ethoxycarbonylethylphosphonic acid.

6. Process for the preparation of the compounds of claim 1, characterised in that an α-aminophosphonate derivative of the general formula II:

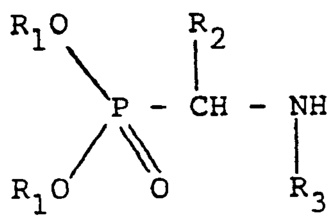

(II)

in which $R_1$, $R_2$ and $R_3$ have the meanings defined in claim 1, is condensed with an excess of β-chloroethyl isocyanate of the formula III:

$Cl-CH_2-CH_2-N=C=O$ (III)

to give derivatives of the general formula IV:

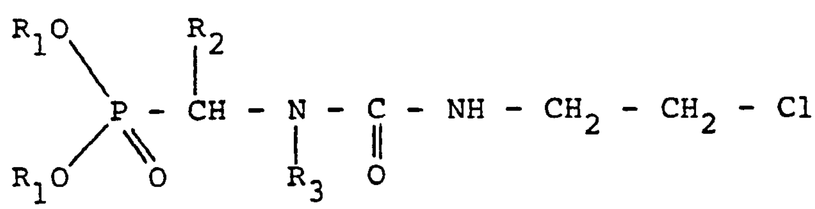

(IV)

in which $R_1$, $R_2$ and $R_3$ have the meanings defined in claim 1, which derivatives are then converted into nitroso compounds by means of a nitrosation agent.

7. Process according to claim 6, characterised in that the compounds of the general formula II are condensed with β-chloroethyl isocyanate III in water or in a chlorinated solvent at a temperature of from 0 to 10°C and in the presence or absence of an organic or mineral base.

8. Process according to claim 6, characterised in that the compounds of the general formula IV are converted into nitroso compounds of the general formula I by means of sodium nitrite in formic acid or by means of nitrosyl chloride in pyridine.

9. Pharmaceutical compositions containing as the active ingredient a compound according to any one of claims 1 to 5 in association or in admixture with a pharmaceutically acceptable, non-toxic, inert excipient or carrier.

10. Compounds corresponding to the general formula IV:

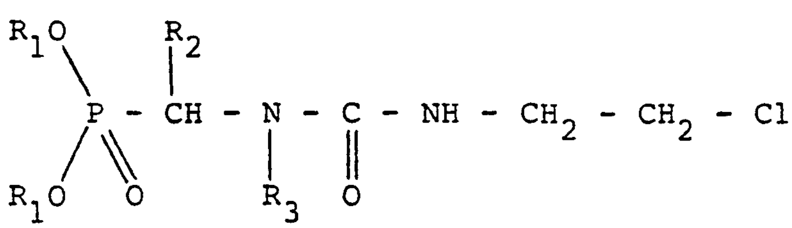

(IV)

in which the symbols $R_1$, $R_2$ and $R_3$ have the same meanings as in claim 1.